Europäisches Patentamt

European Patent Office

Office européen des brevets

(11) Publication number: **0 266 892 B1**

## EUROPEAN PATENT SPECIFICATION

(45) Date of publication of patent specification: **29.01.92**   (51) Int. Cl.⁵: **A61L 15/07**, //C08L75/04

(21) Application number: **87308685.4**

(22) Date of filing: **30.09.87**

(54) Polyurethane casting tape.

(30) Priority: **30.09.86 US 913809**

(43) Date of publication of application:
**11.05.88 Bulletin 88/19**

(45) Publication of the grant of the patent:
**29.01.92 Bulletin 92/05**

(84) Designated Contracting States:
**AT BE CH DE ES FR GB IT LI NL SE**

(56) References cited:
**EP-A- 0 086 621**
**EP-A- 0 221 669**
**US-A- 4 454 873**

(73) Proprietor: **JOHNSON & JOHNSON CONSUM-
ER PRODUCTS, INC.
Grandview Road
Skillman, New Jersey 08558(US)**

(72) Inventor: **Yoon, Hee Kyung
7 Redwood Road
North Brunswick, NJ 08902(US)**
Inventor: **Sun, Robert Li-Jiun
6 Young Drive
Stanhope, NJ 07874(US)**

(74) Representative: **Jones, Alan John et al
CARPMAELS & RANSFORD 43 Bloomsbury
Square
London, WC1A 2RA(GB)**

# Description

The present invention relates to polyurethane casting tape which has controlled tackiness and therefore is better able to be applied to the limb of a patient.

Plaster of Paris casts have been used to immobilize fractured body members or limbs for some time. The plaster of Paris bandages have been supplemented and, to some extent, superseded by synthetic casting tapes or bandages which employ polymeric materials on a woven or knitted substrate. One of these polymeric materials is cured by exposure to ultraviolet light or contain polymers which would cure when reacted with water. Examples of the ultraviolet light cured case can be found in US-A-3,881,473. More recently, water-cured or water-reactive polyurethane compositions have been used in forming orthopedic casting tapes and the polyurethane materials have largely supplemented other polymeric synthetic casting materials. The polyurethane casting materials are of the type which are disclosed in US-A-4,376,438 and US-A-4,441,262.

One of the characteristics of these polyurethane casting materials is the tack or stickiness of the polyurethane prepolymer after the casting tape has been dipped in the water. After contact with water, the polyurethane prepolymers begin to react and as they do, they become quite sticky or tacky. This tackiness is necessary in order that successive layers of the casting tape adhere to each other when the casting tape is wrapped on the limb of the patient. The successive layers of the tape are forced together by applying pressure to the tape by the hands and turning the hands around the limb. This action forces the layers of the tape together and smooths the outside surface of the cast. However, the tackiness causes the prepolymer to adhere to some extent to the gloved hands of the cast technician applying the tape and prevents the hands from sliding over the applied casting tape, making the tapes somewhat difficult to properly apply to the patient. The tackiness may also cause the outer layer of the casting tape to be lifted from the underlying layers as the cast technician attempts to smooth the cast. The most common method of overcoming this problem is to apply a cream or lotion to the hands of the cast technician applying the tape which will overcome this problem to some extent.

It would of course be more desirable if the polyurethane prepolymer composition had controlled tack properties which would avoid the problems encountered in applying the casting tape to the patient.

US-A-4,433,680 and US-A-4,502,479 disclose the use of silicone antifoaming agents in amounts of from 0.1 to 1% in polyurethane cast compositions.

US-A-3,746,680 discloses a plaster of Paris cast which contains a poly(ethylene oxide) which is added to the plaster of Paris to increase the green strength of the casting material.

The present invention provides a polyurethane orthopedic casting tape as defined in claim 1 and a process for preparing such tapes as defined in claim 8 which has controlled tack as compared to prior art casting tapes. The casting tapes of the present invention have a slipperiness or slickness which allows the cast technician or surgeon to smooth the tapes when the tapes are applied to a patient and yet have sufficient tack to adhere the wrapped layers of the tape together. The slipperiness develops after the casting tape has been dipped into water. The controlled tack properties of the casting tapes of the present invention as provided by the incorporation into the prepolymer of from 1 to 10% by weight of a water soluble solid homopolymer of ethylene oxide which is insoluble in the prepolymer and which remains inert in the prepolymer until immersion of the casting tape in water, said homopolymer having a molecular weight of from 100,000 to 4,000,000. The prepolymer may also contain from 1 to 10% by weight of a lubricating fluid. The above weight percents are based on the total weight of the prepolymer. The casting tapes of the present invention are also more comfortable to the body or limbs of the patient and therefore provide better support for the fractured bone. The casting tapes of the present invention have all the other characteristics of polyurethane casting tapes, i.e. they are stronger than plaster of Paris and they are water resistant and X-ray transparent.

The present invention provides a casting tape which has improved application properties which are obtained by the incorporation into the prepolymer of the casting tape a water soluble homopolymer of ethylene oxide. The water soluble polymer is incorporated into the polyurethane prepolymer after the prepolymer is formed. The preferred water soluble polymer is a poly(ethylene oxide) polymer that is a commercially available solid material provided by the Union Carbide Corporation under the trade name "POLYOX" and having a weight average molecular weight from approximately 100,000 to 4,000,000. The water soluble polymers having molecular weights above 3,000,000 have a tendency to develop stringiness between the casting tape and the gloved hands of the cast technician when the cast is wrapped, and casting tapes made with such water soluble polymers are not as esthetically desirable as those made with a water soluble polymer having a molecular weight of 3,000,000 or less. The water soluble polymer is added to the prepolymer in an amount to 1% to 10% based on the total weight of the prepolymer added to the fabric substrate. The water soluble polymer is not soluble in the polyurethane prepolymer but remains inert in the prepolymer until the casting tape is immersed in water prior to application of the tape to the patient. As the water soluble polymers of the present invention are very soluble in water, they dissolve rapidly and provide a slipperiness to the casting tape when the tape is applied to the patient.

In addition to the water soluble polymer it may also be advantageous to add large amounts of a lubricating fluid, such as a mineral oil or a siloxane polymer, to the prepolymer composition which also assists in providing the slickness or slipperiness properties of the polyurethane casting tape of the present invention. The amount of the lubricating fluid added to the prepolymer composition is from 1 to 10% by weight, based on the total weight of the prepolymer composition. The preferred amount of lubricating fluid added is from 2 to 5% by weight, based on the total weight of the composition. The slipperiness provided by the water soluble polymer will diminish as the prepolymer of the casting tape cures. This is because of the elimination of the water in the casting tape due to evaporation and absorption by the cast underpadding. As the water is eliminated, as the water soluble is a solid, it will no longer be available to provide the slipperiness. However, the lubricating fluid is a liquid and remains available to provide slipperiness to finish the smoothing of the cast as the casting tape is setting. The preferred lubricating fluid is a dimethyl siloxane polymer having a viscosity of from 10 to 100,000 centipoise and preferably having a viscosity of 5000 centipoise. If the viscosity of the lubricating fluid is too low, it will tend to separate from the prepolymer formulation. If the viscosity of the lubricating fluid is too high, it is difficult to uniformly blend into the prepolymer formulation. The dimethyl siloxane polymers are available in various viscosities under the trade name DOW CORNING 200 Fluid from the Dow Corning Corporation. Other suitable siloxane polymers are available from Union Carbide under the tradename L-45 and from the General Electric Company under the tradename SF-96. The dimethyl siloxane polymers have been used an anti-foam agents in polyurethane casting tapes but in much smaller amounts (1% or less) than are used in the present invention.

The polyurethane prepolymers which are used in the orthopedic casting tapes of the present invention are the type disclosed in US-A-4,376,438 and US-A-4,411,262. The polyurethane prepolymes disclosed in US-A-4,376,438 contain an amino-polyol which is reacted with an isocyanate. This prepolymer does not need an additional catalyst as the tertiary amine nitrogen in the amino-polyol provides the catalytic activity necessary to cure the prepolymer when the prepolymer is contacted with water. The prepolymer disclosed in US-A-4,411,262 employs a catalyst to activate the prepolymer when the casting tape containing the prepolymer is dipped in water. The preferred catalyst used in the casting tapes of the present invention is the catalyst which is disclosed in US-A-4,433,680 which is a dimorpholinodiethylether (DMDEE).

Generally, the aromatic isocyanates useful in the prepolymer system of the present invention may be any of the aromatic polyisocyanates known in polyurethane chemistry which are described for example in "Polyurethanes Chemistry and Technology." Part I, Interscience Publishers (1962).

The aromatic polyisocyanates preferred include toluene diisocyanate (TDI), such as the 80/20 or the 65/35 isomer mixture of the 2,4 and 2,6 isomeric forms; diphenylmethane diisocyanate (MDI), such as the 4,4′, the 2,4′ and the 2,2′ isomeric forms of isomeric mixtures thereof; modified MDI containing additional groups such as carbodiimide groups, urethane groups and allophanate groups; polymethylene polyphenylisocyanates (Polymeric MDI) which are derived from phosgenation of the condensation products of aniline for formaldehyde. The most preferred polyisocyanate is the carbodiimide containing MDI which is readily available commercially, e.g. Isonate®143L or Rubinate® XI-168.

The polyols useful in the prepolymer system of the present invention include polyether polyols and polyester polyols. The polyether polyols may be prepared by the polymerization of epoxides, such as ethylene oxide, propylene oxide, butylene oxide, tetrahydrofuran, styrene oxide or mixtures thereof in the presence of the catalyst. The polyester polyols include the reaction products of polyhydric alcohols and polybasic carboxylic acids. Instead of free carboxylic acids, the corresponding polycarboxylic acid anhydrides or the corresponding polycarboxylic acid esters of low alcohols or mixtures thereof may be used for preparing the polyesters. Polyesters of lactones, such as ε-caprolactone may also be used. The polyols may be hydrophobic polyols or hydrophilic polyols. The use of these hydrophilic polyols results in increased hydrophilicity of the prepolymer formulation. The increased hydrophilicity of the prepolymer formulation causes the water into which the casting tape is dipped to rapidly migrate or diffuse into the prepolymer. The more rapid diffusion of the water causes the casting tape to harden in a shorter period of time then has heretofore been obtained.

The hydrophilic polyols are generally homopolymers of ethylene oxide or random or block copolymers of ethylene oxide with propylene oxide in which the propylene oxide is present in an amount no more than 80% by weight and preferably no more than 65% by weight thereby resulting in a polyol which has hydrophilic characteristic s and referred herein as a hydrophilic polyol.

Typical hydrophobic polyols used in the polyurethane prepolymer are PLURACOL® P-1010 available from BASF Wyandotte and Poly G 36-232 available from Olin Corporation. Typical hydrophilic polyols are Poly G 55-173 and Poly G 76-120 available from Olin Corporation and CARBOWAX® TPEG-990 available from Union Carbide.

The advantage of using the hydrophilic polyol in the prepolymer is that the water necessary to cure the prepolymer will diffuse more rapidly into a hydrophilic prepolymer than a hydrophobic prepolymer which will more rapidly dissolve the water soluble polymer. The use of the hydrophilic prepolymer also results in a faster setting casting tape.

The preferred polyurethane prepolymer is made from diphenylmethanediisocyanate containing carbodiimide groups. These diisocyanates are reacted with a polyol containing two to three functional groups. The polyols may be diols or triols or mixtures of diols and triols. The preferred polyols are poly(oxypropylene)glycol having a hydroxyl number of 232. The molecular weight of the polyols is usually below 2,000 preferably in the range of 700 to 1,500 and most preferably between 700 and 1,100.

The ratio of the polyisocyanate to the polyol in the prepolymer reaction mixture is best expressed by the equivalent ratio. Equivalent weight is determined by dividing the molecular weight of each particular component by its functionality or number of functional groups in the compound. The equivalent ratio is the ratio of the equivalency of the isocyanate to the polyol. The equivalent ratio in the present system should be between 2:1 to approximately 15:1 equivalents of the polyisocyanate to the polyol and preferably from 2:1 to 10:1. These components are combined so that there is an excess of from 5% to 30% NCO groups in the prepolymer.

The polyurethane prepolymer formulations are coated onto a substrate to form the casting tape.

The substrate employed may be any suitable woven or knitted fabric. These fabrics are generally Raschel knits made from cotton, polyester or fiberglass yarns. The coating levels of the prepolymer on the substrate are generally between 30% and 70% based on the total weight of the casting tape. The coating levels depend to some extent on the nature of the substrate with more prepolymer being employed with the cotton and polyester substrates. Fiberglass is the preferred substrate fabric. The coating levels for a fiberglass substrate are generally from about 35 to 48% preferably from 41 to 44% by weight, based on the total weight of the casting tape.

As indicated above, the preferred lubricating fluid is a dimethyl siloxane polymer. These polymers are available in various viscosities ranging from approximately from 10 to 100,000. The preferred viscosity of the dimethyl siloxane polymer is approximately 5,000. Blends of a 200 centipoise and a 30,000 centipoise dimethyl siloxane polymers may be use to obtain a fluid with a viscosity of approximately 5,000.

As indicated above the water soluble polymer used in the present invention is a solid poly-(ethylene oxide) homopolymer preferably having a molecular weight ranging from 10,000 to 3,000,000. The most preferred poly(ethylene oxide) polymer employed has a molecular weight of approximately 100,000.

The poly(ethylene oxide) polymer may be modified to block the reactivity of the hydroxyl end groups by reacting the poly(ethylene oxide) with a blocking compound such as an acid halide: an acid anhydride: an isocyanate, such as phenyl isocyanate: or a silane such as trimethylchloro silane. The blocking compound would prevent the isocyanate in the prepolymer from reacting with the hydroxyl groups in the water soluble polymer.

It is also advantageous to incorporate small amounts of antioxidants or free radical scavengers in the poly(ethylene oxide) to stabilize the material. Typical additives are acetaminophen and hindered phenol s such as Tetrakis(methylene 3-[3′,5′-di-tert-butyl-4-hydroxyphenyl]-propionate)methane. The preferred method of incorporating the antioxidant into the poly(ethylene oxide) is to dissolve the antioxidant in an organic solvent and coat the poly-(ethylene oxide) particles with the dissolved antioxidant and then dry the coated particles to evaporate the solvent. The poly(ethylene oxide) may also mechanically be treated to reduce the particles of the polymer to a uniform particle size, approximately 100 mesh US (0.149 mm), to prevent any problems in applying the prepolymer to the casting tape substrate. Since the poly(ethylene oxide) is a solid crystalline material, large particles could interfere with the application of the prepolymer to the substrate.

In addition to the polyisocyanates, the polyol and the catalyst the prepolymer reactants may also include a small amount, 0.01% to 1% by weight, of a stabilizer such as benzoyl chloride and a thickening agent such as fumed silica. The use of the thickening agent is to control the viscosity of the prepolymer when it is applied to the cast substrate.

In the following Examples, the effectiveness of the poly(ethylene oxide) in providing the slipperiness is determined by a trained technician measuring the time from the immersion of the casting tape into water to the time of the evidence of slipperiness or tackiness in the tape and intensity of the slipperiness and tackiness on a scale of 0 (no slip or no tack) to 10. Generally, the preferred casting tapes are those which exhibit a slipperiness intensity which is 5 or greater and a tackiness intensity of 5 or less.

The conformability of the casting tapes in the Examples was determined according to the procedure explained on page 234 of the Proceedings of the 10th Annual Meeting of the Society of Biomaterials, April 27 - May 1, 1984. The slipperiness, tackiness and conformability were measured against a commercially available polyurethane fiberglass casting tape sold under the trade name Improved DELTA-LITE® Casting Tape. The substrate used in the tapes of the Examples was the same substrate used in the Improved DELTA-LITE® Casting Tape. The slipperiness intensity of the DELTA-LITE Casting Tape was 0, the tackiness 10 and the conformability 100. The crush strength of the polymer casting tapes was determined as follows: test cylinders were made by wrapping fiver layers of 4 1/2 inch (11.43 cm) wide casting tape around a 2-3/4 inch (6.985 cm) diameter metal dowel. The cylinders were aged for 24 hours at room temperature and their crush strengths were determined. The crush strength of test cylinder samples is determined with a Chatillon compression tester. The samples are compressed a distance of 1 centimeter and the load necessary to deflect the test cylinders determined. Preferably, the crush strength should be greater than 90 pounds (40.824 kg) for a test cylinders aged 24 hours and made with 5 layers of a 4 inch (10.16 cm) wide casting tape on a dowel of this size.

Example I

In a reaction kettle equipped with nitrogen gas inlet, condenser, thermometer and mechanical stirrer was charged 59.172 parts of Isonate 143L, and 0.046 parts of benzoyl chloride. The mixture was stirred for one half hour until a homogeneous mixture was obtained. A polyol mixture made from 12.928 parts of Poly-G 36-232, 19.447 parts of Pluracol P-1010, 1.1044 part of Aerosil 974 and 1.602 parts of DMDEE was added to the reaction kettle. When the exotherm of the reaction had subsided, the mixture was held at a temperature of 50°C for one hour and then cooled to room temperature.

100 parts of urethane polymer thus obtained was blended with 1.8 parts of dimethyl siloxane polymer (DC-200) (Vis. 200 cps), and 3.2 parts of DC-200 (Vis 30,000 cps) and 1.0 parts of POLYOX N-10, (100,000 weight average molecular weight) under a nitrogen atmosphere at room temperature until a homogeneous mixture was obtained.

A casting tape made from this coated prepolymer on a fiberglass substrate was applied as a short leg cast. The slipperiness was detected at 0.5 minutes after immersion in water and had an intensity of 7. Tackiness was detected at 0.4 minutes and had an intensity of 4. The cast set in 5 minutes and had a crush strength of 124 pounds (56.25 kg) and a conformability value of 117.

Example II

This Example shows the procedure for blocking the hydroxyl groups on the poly(ethylene oxide) to provide a more stable product.

To a reaction vessel, equipped with nitrogen inlet, reflux condenser and mechanical stirrer, 37.69 parts of Poly(ethylene oxide) (POLYOX N-3,000) (400,000 weight average molecular weight) was slowly added to a solution consisting of 251.31 parts of Dow Corning silicone fluid (5,000 cps) and 2.51 parts of SILWET L-7602 as a surfactant. The mixture was stirred at room temperature until a stable uniform suspension was obtained. To the uniform suspension 0.02021 parts of phenylisocyanate was then added to block the hydroxyl groups of the poly(ethylene oxide). The temperature was then raised to 80°C and kept at 80°C for six hours. The heat was removed and the stirring was continued overnight. The product thus obtained should be used promptly as the product tends to separate into different phases. A casting tape was made using the polyurethane prepolymer of Example I to which 6% by weight of the blocked poly(ethylene oxide) and silicone fluid mixture obtained above was added. A casting tape made from this prepolymer was aged for 14 days at 50°C. The aged casting tape was used to form a short leg cast. Slipperiness was detected at 1.3 minutes and had an intensity of 5. Tackiness was detected at 0.3 minutes and had an intensity of 5. The casting tape had a conformability value of 118 and a crush strength of 135 pounds (61.24 kg).

Example III

To a reaction vessel equipped with nitrogen gas inlet, condenser, thermometer and mechanical stirrer was charged 547.2 parts of Isonate 143L, 26.83 parts of Dow Corning 200 Fluid (200 cps), 8.94 parts of Dow Corning 200 fluid (30,000 cps), and 0.44 parts of benzoyl chloride. The mixture was stirred for one half hour until a homogeneous mixture was obtained. A polyol mixture made from 198.0 parts of TPEG-990, 129.71 parts of Poly G-55-173, 10.94 parts of Aerosil 974 and 8.75 parts of DMDEE was added to the reaction kettle. When the exotherm of the reaction had subsided, the mixture was held at 50°C for one hour. The mixture was then cooled down to 35°C and 7.22 parts of POLYOX-N-3,000 (400,000 weight average molecular weight) was added to the reactor and the mixture continued mixing for an additional two hours without heating.

The prepolymer was coated on a fiberglass substrate to form a casting tape. The casting tape was used to form a short leg cast. Slipperiness was detected at 0.5 minutes and had an intensity of 7. Tackiness was detected at 0.4 minutes and had an intensity of 4. The cast had a set time of 4.5 minutes and had a crush strength of 104 pounds (47.17 kg) and a conformability value of 125.

Example IV

In a 5 liter reaction flask equipped with nitrogen gas inlet, condenser, thermometer and a mechanical stirrer was charged 1831.68 parts of Isonate 143L, 78.24 parts of Dow Corning 200 (200 cps) fluid, and 1.30 parts of benzoyl chloride. While stirring, a polyol mixture made from 381.65 parts of Poly G 35x-280, 381.65 parts of Carbowax and 32.44 parts of DMDEE was added to the mixture at room temperature until the exotherm had subsided. Then the mixture was held at 50°C for one hour. After the mixture was cooled to 35°C, 12.98 parts of POLYOX-N 12K (1,000,000 weight average molecular weight) was then added to the prepolymer and the mixture was continued to be stirred for an additional two hours at room temperature.

The prepolymer was coated on a fiberglass substrate to form a casting tape. The casting tape was used to form a short leg cast. Slipperiness was detected at 0.6 minutes and had an intensity of 10. Tackiness was detected at 0.2 minutes and had an intensity of 5. The cast had a set time of 4.5 minutes and a conformability value of 103.

## Claims

1. A casting tape comprising a water curable polyurethane prepolymer formulation coated on a fabric substrate, said prepolymer formulation having controlled tackiness and containing from 1 to 10 percent by weight, based on the total weight of the formulation, of a water soluble solid homopolymer of ethylene oxide which is insoluble in the prepolymer and which remains inert in the prepolymer until immersion of the casting tape in water, said homopolymer having a molecular weight of from 100,000 to 4,000,000.

2. The casting tape of claim 1 in which the molecular weight of the water soluble polymer is between 1,000,000 and 3,000.000.

3. The casting tape of claim 1 or claim 2 in which the prepolymer formulation also contains from 1 to 10 percent by weight, based on the total weight of the formulation, of a lubricating fluid.

4. The casting tape of claim 3 in which the prepolymer formulation contains from 2 to 5 percent by weight of the lubricating fluid.

5. The casting tape of claim 3 or claim 4 in which the lubricating fluid has a viscosity of from 200 to 30,000 centipoise.

6. The casting tape of claim 5 in which the lubricating fluid has a viscosity of 5,000 centipoise.

7. The casting tape of any one of claims 3 to 6 in which the lubricating fluid is a dimethyl siloxane polymer.

8. A process for preparing a casting tape which comprises (1) incorporating in a water curable polyurethane prepolymer formulation, said prepolymer formulation having controlled tackiness, from 1 to 10 percent by weight, based on the total weight of the formulation, of a water soluble solid homopolymer of ethylene oxide which is insoluble in the prepolymer and which remains inert in the prepolymer until immersion of the casting tape in water, said homopolymer having a molecular weight of from 100,000 to 4,000,000, and (2) coating said formulation on a fabric substrate.

9. A process according to claim 8 in which the molecular weight of the water soluble homopolymer is between 1,000,000 and 3,000,000.

10. A process according to claim 8 in which the casting tape also includes a lubricating fluid as defined in and in the amounts defined in any one of claims 3 to 7.

## Revendications

1. Un bandage orthopédique comprenant une formulation de pré-polymère à base de polyuréthanne pouvant être cuit dans l'eau enduit sur un substrat de tissu, ladite formulation de prépolymère ayant un caractère poisseux contrôlé et contenant de 1 à 10 pour-cent en masse par rapport à la masse totale de la formulation, d'un homopolymère solide, soluble dans l'eau, d'oxyde d'éthylène qui demeure non soluble dans le pré-polymère et qui demeure à l'état inerte dans le pré-polymère jusqu'à ce qu'on trempe le bandage orthopédique dans l'eau, ledit homopolymère ayant une masse moléculaire allant de 100.000 à 4.000.000.

2. Le bandage orthopédique de la revendication 1, dans lequel la masse moléculaire du polymère soluble dans l'eau est comprise entre 1.000.000 et 3.000.000.

3. Le bandage orthopédique de la revendication 1 ou de la revendication 2, dans lequel la formulation de pré-polymère contient également de 1 à 10% en masse, par rapport à la masse totale de la formulation, d'un fluide lubrifiant.

4. Le bandage orthopédique de la revendication 3, dans lequel la formulation de pré-polymère contient de 2 à 5 pour-cent en masse du fluide lubrifiant.

5. Le bandage orthopédique de la revendication 3 ou de la revendication 4, dans lequel le fluide lubrifiant a une viscosité allant de 200 à 30.000 centipoises.

6. LA bandage orthopédique de la revendication 5 dans lequel le fluide lubrifiant a une viscosité de 5.000 centipoises.

7. Le bandage orthopédique de l'une des revendications 3 à 6, dans lequel le fluide lubrifiant est un polymère de diméthyl siloxane.

8. Un procédé pour préparer un bandage orthopédique qui comprend (1) le fait d'incorporer dans une formulation de pré-polymère à base de polyuréthanne pouvant être cuit dans l'eau, ladite formulation de pré-polymère ayant un caractère poisseux contrôlé, de 1 à 10 pour cent en masse, par rapport à la masse totale de la formulation, d'un homopolymère solide, soluble dans l'eau, d'oxyde d'éthylène qui est non soluble dans le pré-polymère et qui demeure à l'état inerte dans le pré-polymère jusqu'à ce que le bandage orthopédique soit trempé dans l'eau, ledit homopolymère ayant une masse moléculaire allant de 100.000 à 4.000.000 et, (2) le fait d'enduire ladite formulation sur un substrat de tissu.

9. Un procédé selon la revendication 8, dans lequel la masse moléculaire de l'homopolymère soluble dans l'eau est comprise entre 1.000.000 et 3.000.000.

10. Un procédé selon la revendication 8, dans lequel le bandage orthopédique comprend également un fluide lubrifiant comme défini et dans les quantités définies dans l'une des revendications 3 à 7.

**Patentansprüche**

1. Orthopädische Bandage mit einem Gehalt an einer durch Wasser aushärtbaren Polyurethanpräpolymerformulierung, welche als Überzug auf einem Stoffsubstrat aufgebracht ist, und welche Präpolymerformulierung eine geregelte Klebrigkeit aufweist und 1 bis 10 Gew.-%, bezogen auf das Gesamtgewicht der Formulierung, von einem wasserlöslichen, festen Homopolymer von Ethylenoxid enthält, welches Polymer in dem Präpolymer unlöslich ist und bis zum Eintauchen der orthopädischen Bandage in Wasser in dem Präpolymer inert verbleibt, und welches Homopolymer ein Molekulargewicht von 100.000 bis 4,000.000 hat.

2. Orthopädische Bandage nach Anspruch 1, wobei das Molekulargewicht des wasserlöslichen Polymers zwischen 1,000.000 und 3,000.000 beträgt.

3. Orthopädische Bandage nach Anspruch 1 oder 2, wobei die Präpolymerformulierung auch 1 bis 10 Gew.-%, bezogen auf das Gesamtgewicht der Formulierung, einer Schmiermittelflüssigkeit enthält.

4. Orthopädische Bandage nach Anspruch 3, wobei die Präpolymerformulierung 2 bis 5 Gew.-% der Schmiermittelflüssigkeit enthält.

5. Orthopädische Bandage nach Anspruch 3 oder 4, wobei die Schmiermittelflüssigkeit eine Viskosität von 200 bis 30.000 cP aufweist.

6. Orthopädische Bandage nach Anspruch 5, wobei die Schmiermittelflüssigkeit eine Viskosität von 5.000 cP hat.

7. Orthopädische Bandage nach einem der Ansprüche 3 bis 6, wobei die Schmiermittelflüssigkeit ein Dimethylsiloxanpolymer ist.

8. Verfahren zur Herstellung einer orthopädischen Bandage, welches umfaßt: (1) die Einverleibung eines wasserlöslichen, festen Homopolymers von Ethylenoxid in eine in Wasser aushärtbare Polyurethanpräpolymerformulierung mit geregelter Klebrigkeit, in einer Menge von 1 bis 10 Gew.-%, bezogen auf das Gesamtgewicht der Formulierung, welches Homopolymer in dem Präpolymer unlöslich ist, und welches Homopolymer in dem Präpolymer bis zum Eintauchen der orthopädischen Bandage in Wasser inert bleibt, wobei dieses Homopolymer ein Molekulargewicht von 100.000 bis 4,000.00 aufweist; und (2) das Aufbringen dieser Formulierung auf ein Stoffsubstrat.

9.  Verfahren nach Anspruch 8, wobei das Molekulargewicht des wasserlöslichen Homopolymers zwischen 1,000.000 und 3,000.000 beträgt.

10. Verfahren nach Anspruch 8, wobei die orthopädische Bandage auch eine Schmiermittelflüssigkeit enthält, wie dieselbe in einem der Ansprüche 3 bis 7 definiert worden ist, und in den dort angegebenen Mengen.